(19) Europäisches Patentamt
European Patent Office
Office européen des brevets

(11) **EP 3 748 245 B1**

(12) **EUROPEAN PATENT SPECIFICATION**

(45) Date of publication and mention
of the grant of the patent:
**09.08.2023  Bulletin 2023/32**

(21) Application number: **19178328.1**

(22) Date of filing: **05.06.2019**

(51) International Patent Classification (IPC):
**F24F 11/38** (2018.01)    **F28G 1/16** (2006.01)
**F28G 9/00** (2006.01)    **F28G 13/00** (2006.01)
**F24F 110/10** (2018.01)    **F24F 110/20** (2018.01)

(52) Cooperative Patent Classification (CPC):
**F24F 11/38; F28G 1/166; F28G 9/00; F28G 13/00;**
F24F 2110/10; F24F 2110/20; F28G 2015/006

(54) **CLEANING SYSTEM FOR AIR CONDITIONING SYSTEM, HEAT EXCHANGER ASSEMBLY AND METHODS**

REINIGUNGSSYSTEM FÜR KLIMAANLAGE, WÄRMETAUSCHERANORDNUNG UND VERFAHREN

SYSTÈME DE NETTOYAGE POUR SYSTÈME DE CLIMATISATION, ENSEMBLE D'ÉCHANGEUR DE CHALEUR ET PROCÉDÉS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**09.12.2020  Bulletin 2020/50**

(73) Proprietor: **Robert Bosch GmbH**
**70442 Stuttgart (DE)**

(72) Inventors:
• **Cola, Fabrizio**
**534635 Singapur (SG)**
• **Soon, Hwee Ping**
**573943 Singapur (SG)**
• **Pradipta, Justin**
**570109 Singapur (SG)**
• **Krishnaswamy, Mohan**
**573943 Singapur (SG)**

(56) References cited:
WO-A1-2017/059119    CN-U- 202 581 563
JP-A- 2000 111 076    KR-B1- 100 526 446

**Description**

**TECHNICAL FIELD**

The present invention relates to a cleaning system for cleaning a heat exchanger of an air conditioning system, a heat exchanger assembly and an air conditioning system including the cleaning system, a method for operating a cleaning system and a method for determining the useful life of the heat exchanger.

**BACKGROUND**

Air conditioning systems may be configured to condition air by adjusting at least one of the following air properties: temperature and humidity. Thus, air conditioning systems may be configured to provide at least one of: heating, cooling, decreasing humidity, increasing humidity.

Existing cleaning methods of air conditioning systems address only the problem of removal of larger impurities by the use of a water spray nozzle. Spreading of bacteria and black mould, a known problem of indoor air conditioning heat exchangers working in dehumidification mode, cannot be prevented with this solution alone. Thus, periodic cleaning routines are usually performed on the heat exchanger assemblies. These may be time and cost intensive.

Therefore, there is a desire to simplify and improve cleaning processes for air conditioning systems.

KR 100 526 446 B1 discloses an air cleaning and ventilation heat recovery system provided to efficiently recover heat energy while cleaning air supplied to inside with reducing pressure loss by using a surface of a heat exchanger as a dust collector of an air cleaner. A rotary total heat exchanger is mounted at a certain point of a ventilation duct discharging air from the inside to the outside and an air supply duct supplying air to the inside from the outside. A high voltage charging device is installed at an upstream of the total heat exchanger mounted at the air supply duct. An ultra violet lamp is installed at the upstream of the total heat exchanger mounted at the air supply duct. A cleaning water spray device cleans alien substances attached to the total heat exchanger.

**SUMMARY**

It is therefore, an object of the invention to provide a cleaning system for cleaning a heat exchanger of an air conditioning system. It is a further object of the invention to provide an improved heat exchanger assembly for the air conditioning system. It is a further object of the invention to provide an improved air conditioning system. It is a further object of the invention to provide an improved method for operating a cleaning system for cleaning the heat exchanger. It is a further object of the invention to provide a method of determining the useful life of a heat exchanger of an air conditioning system.

Various embodiments relate to a cleaning system for cleaning a heat exchanger of an air conditioning system. The cleaning system includes a spray subsystem configured to, during a cleaning cycle, spray a cleaning liquid onto the heat exchanger. The cleaning system further includes a UV light source. The UV light source is configured to irradiate a surface of the heat exchanger, during the cleaning cycle. The UV light source may be used for reducing a biofilm. The cleaning system includes a sensor subsystem for monitoring an effectiveness of the heat exchange between the heat exchanger and air. A controller is provided, configured to control the cleaning system to clean the heat exchanger, and further configured to obtain sensor data from the sensor subsystem. The controller is configured to activate a next cleaning cycle when the effectiveness has deviated from a pre-determined threshold.

Various embodiments relate to a heat exchanger assembly for an air conditioning system including a cleaning system as described in the present disclosure.

Various embodiments relate to an air conditioning system configured to condition air by providing one or more of: heating, cooling, reducing humidity, increasing humidity. The air conditioning system includes the cleaning system as described herein, or the heat exchanger assembly as described herein. The operation(s) may be processed via control by the controller.

Various embodiments relate to a method for operating a cleaning system as described herein. The method includes operating the spray subsystem to spray a cleaning liquid onto the heat exchanger, and operating the UV light source to irradiate a surface of the heat exchanger.

Various embodiments relate to a method for determining a useful life of a heat exchanger of an air conditioning system as described in the present disclosure. The method includes providing a history log of effectiveness of the heat exchange between the heat exchanger and air. The history log includes data obtained from a sensor subsystem. The method further includes determining an effect of a latest cleaning cycle, and thereby evaluating an estimated useful life of the heat exchanger.

## BRIEF DESCRIPTION OF THE DRAWINGS

In the following description, various embodiments of the present invention are described with reference to the following drawings, in which:

FIG. 1 shows a schematic cross section view of an air conditioning system 100, in accordance with various embodiments;
FIG. 2 shows another schematic cross section view of an air conditioning system 100, showing a sensor subsystem 130 in accordance with various embodiments;
FIG. 3 shows another schematic cross section view of an air conditioning system 100, showing a protective screen 129 disposed to at least partially block UV light emitted by the UV light source 128, in accordance with various embodiments.

## DETAILED DESCRIPTION

In the description which follows, the drawing figures are not necessarily to scale and certain features may be shown in generalized or schematic form in the interest of clarity and conciseness or for informational purposes.

As used herein, the term "and/or" includes any and all combinations of one or more of the associated listed items.

The cleaning system as described herein is suitable for heat exchanger assemblies and consequently, air conditioning systems including such heat exchanger assemblies. The heat exchanger assemblies are configured to condition air by providing one or more of: heating, cooling, reducing humidity, increasing humidity. References in the present disclosure to "humidity" may refer to relative humidity of air.

A heat exchanger according to various embodiments may be a coil, for example, a condenser coil or an evaporator coil. For example, compressed liquid refrigerant may flow through a tubing, and before entering an evaporator coil, it may passes through an expansion valve. When the refrigerant expands by passing through the expansion valve, the refrigerant is cooled and absorbs heat from the air surrounding the coil. In this example, refrigerant and air are separated by the walls of the coil tubing. In one example the heat exchanger may be a heating, ventilation, and air conditioning (HVAC) air coil. In another example, the coil may be configured to receive hot or cold water externally supplied, and to remove heat from or add heat to surrounding air through heat transfer. Coils may be made, e.g., from copper or aluminum tubing.

A cleaning system for cleaning the heat exchanger is provided in accordance with various embodiments. The cleaning system includes a spray subsystem configured to, during a cleaning cycle, spray a cleaning liquid onto the heat exchanger. The cleaning system further includes a UV light source. The UV light source is configured to irradiate a surface of the heat exchanger, during the cleaning cycle, for reducing biofilm. The spray subsystem may be used for cleaning of the heat exchanger by removing larger particles, through the use of liquid sprays, for example water sprays. The liquid may be feed from a reservoir. The reservoir may receive used liquid for re-use. The UV light source may sterilize the surface of the heat exchanger which is irradiated with UV, for example removing and/or degrading biofilms generated by growing bacteria and/or moulds. Continuous recirculation of water at ambient temperature, which may be provided, e.g. by the spray subsystem, can lead to an increased risk of bacteria and mould growth. This could worsen the indoor air quality experienced by the occupants and affect their wellbeing. The destruction of biofilms on the heat exchanger's surface by irradiation from the UV light source may counter these effects and restore the air conditioning performance and effectiveness which may have been worsen by biofilms.

Various embodiments relate to a method for operating a cleaning system as described herein. The method includes operating the spray subsystem to spray a cleaning liquid onto the heat exchanger, and operating the UV light source to irradiate a surface of the heat exchanger. For example, the UV light source may be operated first, to destroy biofilms and the spray subsystem may be operated after the UV light source to remove destroyed biofilm.

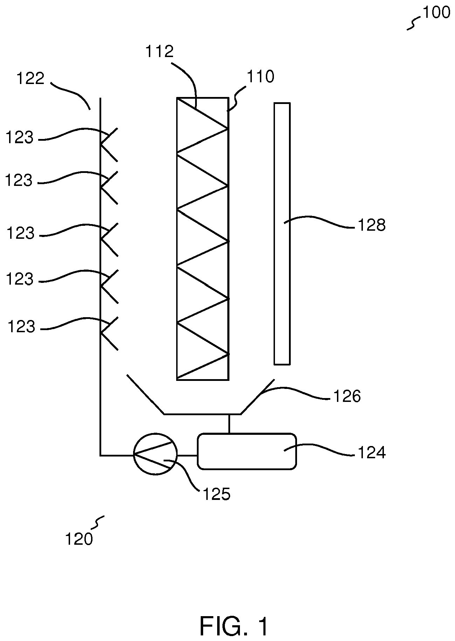

FIG. 1 shows a schematic cross section view of an air conditioning system 100, in accordance with various embodiments. The air conditioning system 100 may include the heat exchanger assembly 110 for receiving air flow. The heat exchanger assembly may include the heat exchanger 112. The air conditioning system 100 includes the cleaning system 120 for cleaning the heat exchanger 112.

The cleaning system 120 includes a spray subsystem 122 configured to, during a cleaning cycle, spray a cleaning liquid onto the heat exchanger 112. The spray subsystem is configured and arranged such that liquid may be sprayed, for example from spray nozzles 123 onto a surface of the heat exchanger. Accordingly, the spray subsystem 122 may include spray nozzles 123 arranged, in relation to the heat exchanger, such that the cleaning liquid is sprayable onto the surface of the heat exchanger 112.

According to various embodiments, the cleaning system 120 may further include a cleaning liquid reservoir 124. The cleaning system may further include a cleaning liquid pump 125. The cleaning liquid reservoir 124 may be in fluid connection with the spray nozzles 123 via the cleaning liquid pump 125.

According to various embodiments, the cleaning system 120 may further include a cleaning liquid collection tray 126 disposed in proximity to the heat exchanger 112 to collect the cleaning liquid, optionally, wherein the cleaning liquid collection tray 126 is configured to return the cleaning liquid to the cleaning liquid reservoir 124.

The spray subsystem 122 may be used for the removal of larger particles. The spray subsystem 122 may also be used for removal of residues of a destroyed biofilm. The spray subsystem 122 may include one or more spray nozzles 123. The spray nozzles 123 may be arranged as an array of spray nozzles. The spray nozzles 123 may be arranged in relation to the heat exchanger 112 such that a substantial surface of the heat exchanger 112 may be sprayed with liquid. For example, different spray array geometries may cover the spraying of a substantially entire surface area of different heat exchanger 112 geometries.

Liquid may be supplied to the spray nozzles 123 by a cleaning liquid reservoir 124 and a cleaning liquid pump 125. The cleaning liquid pump 125 may be configured to pressurize the liquid stream, for example, to a pressure of 10 bar or above. The pressure is preferably within the range of 5 bar to 15 bar, endpoints included. The pump 125 may be a micropump. A cleaning liquid collection tray 126 may be used to recover the liquid and may further be configured to return the liquid to the cleaning liquid reservoir 124. The liquid may be water. For an indoor heat exchanger working in cooling mode, the water produced by dehumidification can be recovered as well to supplement liquid (e.g. water) used for filling the cleaning liquid reservoir. For an outdoor heat exchanger working in condenser mode, the water reservoir may be without a supplementary source for its filling.

The cleaning system 120 further includes a UV light source 128 configured to irradiate a surface of the heat exchanger 112, during the cleaning cycle, for reducing biofilm. Thus, the UV light source 128 is arranged in relation to the heat exchanger such that, during operation, the surface of the heat exchanger 112 is irradiated with UV light. The UV light source 128 may be a UVC light source. The UVC light source 128 may have UV emission (for example a UV emission peak) in the range from 200 nm to 280 nm, for example in the range from 250 nm to 280 nm, for example 254 nm. The UVC light source 128 may be a mercury-based lamp. The UVC light source 128 may be powered by the local electricity grid.

The cleaning system 120 may further include a protective screen 129 disposed to at least partially block UV light emitted by the UV light source 128 in a direction other than towards the heat exchanger 112. The UV light source 128 may be sized and positioned to face the heat exchanger 112 only, and optionally other components of the heat exchanger assembly 110 which are not sensitive to UV light, and not interact with other components of the heat exchanger assembly 110 and other components of the air conditioning system 100. In particular sensible areas of the heat exchanger assembly 110 and other components of the air conditioning system 100, such as plastic components, may be shielded from the UV light by the protective screen 129.

For an indoor heat exchanger working in cooling mode, the use of the UV light source may remove the biofilm and improve the air quality for the occupants. For an outdoor heat exchanger working in condenser mode, UV light would not substantially affect the air quality directly. However, the removal of the biofilm may improve the heat exchanger effectiveness and produced benefits in terms of energy savings.

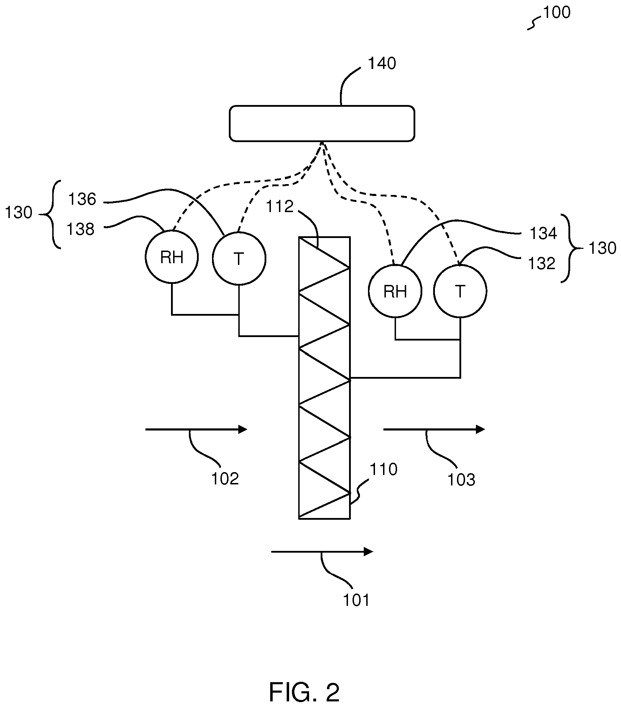

FIG. 2 shows another schematic cross section view of an air conditioning system 100, showing a sensor subsystem 130 in accordance with various embodiments.

The cleaning system 120, according to various embodiments, further includes a sensor subsystem 130 for monitoring an effectiveness of the heat exchange between the heat exchanger 112 and air and/or of the air conditioning system 100. The sensor subsystem 130 may include a downstream temperature sensor 132 disposed in relation to the heat exchanger 112 such that a downstream air temperature is measurable. The sensor subsystem 130 may include an upstream temperature sensor 136 disposed in relation to the heat exchanger 112 such that an upstream air temperature is measurable. In FIG. 2, the direction of air flow is represented by arrow 101, the upstream is represented by arrow 102 and the downstream is represented by arrow 103. Downstream air may be air which has passed through the heat exchanger 112, and may also be named outlet air. Upstream air may be air which is yet to pass through the heat exchanger 112, and may also be named inlet air. The sensor subsystem 130 may include a downstream humidity sensor 134 disposed in relation to the heat exchanger 112 such that a downstream air humidity is measurable. The sensor subsystem 130 may include an upstream humidity sensor 138 disposed in relation to the heat exchanger 112 such that an upstream air humidity is measurable. In some embodiments, more or less sensors may be implemented, for example the sensor subsystem 130 may be free of an upstream humidity sensor, and for example, the upstream humidity (if required) may be assumed to be a constant, for example 100%.

According to various embodiments, the air conditioning system 100, for example the sensor subsystem 130, may include a refrigerant temperature sensor, for measuring the refrigerant temperature in the heat exchanger, or a value corresponding thereto.

The air conditioning system 100, according to various embodiments, further includes a controller 140 configured to control the cleaning system 120 to clean the heat exchanger 112, and further configured to obtain sensor data from the sensor subsystem 130 to determine the effectiveness of at least one of: the heat exchanger 112, the heat exchanger assembly 110, and the air conditioning system 100. The effectiveness of the heat exchanger 112, the heat exchanger assembly 110, and the air conditioning system 100 may all be proportional to each other, and may be substantially the same. According to the invention, the cleaning system 120 includes the controller 140. In examples not falling under the scope of the invention, the controller 140 may be external to the cleaning system 120, however the controller 140 may still be included in the air conditioning system 100 and may be operable to communicate with the sensor subsystem 130 of the cleaning system 120.

According to various embodiments, the controller 140 is configured to monitor the effectiveness, of the heat exchanger 112 and/or the heat exchanger assembly 110 and/or of the air conditioning system 100 when the air conditioning system 100 is in operation, in a continuous or in a time-discrete monitoring pattern. Continuous monitoring may be used to evaluate the optimal time or frequency for performing the heat exchanger cleaning. This may ensure that an optimal air quality is achieved with a minimum additional energy consumption, and at the same time extend the life of the cleaning system 120 by minimizing run time.

According to some embodiments, the term "continuously" may mean that the effectiveness is repeatedly monitored, for example sensor data from the sensor subsystem is acquired at a certain rate of acquisitions per a fixed time period, for example, at (or at a higher rate) rate of 1 acquisition per minute, at (or at a higher rate) rate of 1 acquisitions per 10 seconds, at (or at a higher rate) rate of 1 acquisition per second. According to some embodiments, continuous monitoring may mean that after sensor data from the sensor subsystem is acquired, for example, to determine the effectiveness, a next acquisition of sensor data may start, for example as soon as the controller has enough resources to initiate the next acquisition. In one example, the time between two consecutive acquisitions (which may exclude the time required for an acquisition) may be less than 1 minute, for example less than 10 seconds, or less than 1 second. The term "continuous" may mean uninterrupted.

According to various embodiments, a monitoring with a time-discrete monitoring pattern may mean that sensor data from the sensor subsystem is acquired with a predefined time interval, for example, to determine the effectiveness, a next acquisition of sensor data may start, for example, after a certain time has passed, for example, the controller may be configured to trigger acquisitions at fixed times according to a clock. In one example, the time between two consecutive acquisitions may more than 1 minute, for example more than 10 minutes. In another example, the acquisition may be carried out every 2 (or more) minutes according to a clock, for example every 10 (or more) minutes.

According to various embodiments, the effectiveness may be determined, monitored and/or evaluated, at a same time interval or rate than the acquisition of the sensor data, for example as detailed above.

According to various embodiments, the effectiveness of the air conditioning system may be determined with a relation to the effectiveness of the heat exchanger (i.e., the effectiveness of the heat exchange between the heat exchanger and air), for example the effectiveness of the air conditioning system may be determined by applying a polynomial function, such as a linear function for which constants may be predetermined, on the effectiveness of the heat exchanger. For example the effectiveness of the air conditioning system may be proportional to the effectiveness of the heat exchanger by a pre-determined constant of proportionality. In a simple approximation, the constant of proportionality is 1.

According to various embodiments, the controller 140 may be configured to activate a next cleaning cycle when the effectiveness of the heat exchanger 112 and/or of the air conditioning system 100 has deviated from a nominal value. For example, the nominal value may be a pre-determined threshold, for example, a nominal value. When the effectiveness deviates, for example, decreases from above the nominal value to below the nominal value, the controller 140 may start a cleaning cycle (the next cleaning cycle). Instead of the nominal value, an effectiveness of the heat exchanger in its new state or of the air conditioning system in its new state may be used for determining if the respective effectiveness has deviated from a nominal value.

Operation of the cleaning subsystem and the UV light source 128 may be optimized with a control algorithm that monitors the heat exchanger 112 effectiveness and/or the air conditioning system 100 effectiveness in a continuous or in a time-discrete monitoring pattern. The control algorithm may be carried out by the controller 140. The temperature and the humidity sensors (one, some, or all of the sensors) may be used to monitor the air such as the downstream air and/or the upstream air. By combining the data with the sensors reading from the air conditioning system 100, the real time heat exchanger 112 effectiveness and/or the air conditioning system 100 effectiveness may be calculated and compared with the nominal value, for example as expected from a clean heat exchanger. A decision algorithm may then determine the best cleaning routine to follow in order to avoid excessive degradation of the heat exchanger's 112 performance. The decision algorithm and the cleaning routine may be part of the control algorithm.

According to various embodiments, the controller 140 may be configured to compare the effectiveness (of the heat exchanger 112 and/or of the air conditioning system 100) with a previous effectiveness. The effectiveness is determined, for example, the effectiveness may be the latest determined. The previous effectiveness is determined after a previous cleaning cycle, for example within 24 h of the completion of the previous cleaning cycle, such as 30 min after the completion of the previous cleaning cycle. The controller 140 may be configured to activate a next cleaning cycle when the effectiveness has deviated from the previous effectiveness by a predetermined value. The comparison of the effectiveness with a previous effectiveness may be implemented in any suitable manner, for example: as a subtraction, a ratio, or a percentage. Detecting changes in this heat exchanger cleaning output may be used to evaluate the useful life of the heat exchanger and/or the air conditioning system and estimate an optimal time for overhauling.

According to various embodiments, the effect of the cleaning may be measured by evaluating the improvement in the effectiveness before and after each cleaning cycle. The evaluation of this effect may be implemented in any suitable manner, for example: as a subtraction, a ratio, or a percentage of the effectiveness before to the effectiveness after each cleaning cycle.

According to various embodiments, the effectiveness (of the heat exchanger 112 and/or of the heat exchanger assembly 110 and/or of the air conditioning system 100) is stored in the form of a history log. The controller 140 may be configured to, based on a comparison of the effectiveness with the previous effectiveness stored in the history log, evaluate an estimated useful life of the cleaning system and/or of the heat exchanger assembly and/or of the heat exchanger, for example by determining the effect of the cleaning cycle, or the deviation of the effectiveness from the nominal value. Some embodiments relate to a method for determining a useful life of the cleaning system and/or of the heat exchanger 112 for an air conditioning system 100. The method includes providing a history log of effectiveness, and determining the effect of the latest cleaning cycle, and evaluating an estimated useful life of at least one of the cleaning system 120, the heat exchanger 112, the heat exchanger assembly 110, and the air conditioning system 100. In examples not falling under the scope of the invention, instead of storing values corresponding to the effectiveness, data obtained from the sensor subsystem, for example, measurements obtained from one or more of: the upstream temperature sensor, the downstream temperature sensor, the upstream humidity sensor, and the downstream humidity sensor, may be stored in the history log, and the effectiveness may be determined at a later stage, for example, when estimating the useful life of at least one of the cleaning system 120, the heat exchanger 112, the heat exchanger assembly 110, and the air conditioning system 100.

In addition, calculated values may be stored, such as one or more of: upstream air enthalpy, downstream air enthalpy, real air enthalpy change, and ideal air enthalpy change.

By recording a history of these values, consistency in the cleaning activity performance can be measured, and with this an evaluation of the useful life of at least one of: the cleaning system, the heat exchanger assembly, the heat exchanger, and the air conditioning system can be made. This provides an additional measure to reduce the risk of large system failures and provide an optimal indication for the eventual overhaul of the system. According to various embodiments, the history log may be stored in the controller 140. The controller 140 may be an electronic circuit, for example, including a microcontroller and a memory.

According to various embodiments, the controller 140 may control the power, for example an on/off state, of the UV light source 128.

In one example, according to various embodiments, the effectiveness may be determined as a ratio ($\varepsilon$) of the real air enthalpy change ($h_{air,in,real}$ - $h_{air,out,real}$) to the ideal air enthalpy change ($h_{air,in,real}$ - $h_{air,out,ideal}$). The real air enthalpy change may be calculated by the difference between the upstream air enthalpy ($h_{air,in,real}$) and the downstream air enthalpy ($h_{air,out,real}$). The upstream air enthalpy ($h_{air,in,real}$) may be determined as a function of the temperature and humidity of the upstream air, for example at the inlet of the heat exchanger. The downstream air enthalpy ($h_{air,out,ideal}$) may be determined as a function of the temperature and humidity of the downstream air, for example at the outlet side of the heat exchanger. The ideal enthalpy change may be calculated by using the same value of upstream air enthalpy calculated for the real case ($h_{air,in,real}$), while the downstream ideal air enthalpy ($h_{air,out,ideal}$) may be calculated assuming downstream air at a constant humidity, for example, 100% relative humidity, and a temperature equal to the refrigerant temperature in the heat exchanger. An exemplary formula is as follows:

$$\varepsilon = (h_{air,in,real} - h_{air,out,real})/(h_{air,in,real} - h_{air,out,ideal}),$$

with enthalpies calculated, for example, as follows:

$$h_{air,in,real} = f(T_{air,in}, RH_{air,in}), h_{air,out,real} = f(T_{air,out}, RH_{air,out}), h_{air,out,ideal} =$$
$$f(T_{refrigerant}, 100\%),$$

wherein $T_{air,in}$ represents the upstream air temperature, $T_{air,out}$ represents the downstream air temperature, $RH_{air,in}$ represents the upstream air humidity, and $T_{refrigerant}$ represents the temperature of the refrigerant.

The cleaning system 120 according to various embodiments may adopt a predictive algorithm to accomplish the above. The predictive algorithm may be implemented in the controller 140.

In one example, the algorithm evaluates the (current) effectiveness in a continuous or in a time-discrete monitoring pattern, for example as previously explained using the ratio ($\varepsilon$), so that a history of the heat exchanger performance may be recorded. A threshold value can be set to determine when the heat exchanger reaches a state of unacceptable performance. This threshold can be set either as a percentage of a maximum measured effectiveness at the initial heat exchanger operation, or as an absolute difference between the maximum measured effectiveness, or as a fixed value independent of the maximum effectiveness recorded. Multiple thresholds can be set in the same manner to identify different grades of the heat exchanger health, so that information on the relative state of two or more heat exchangers (or heat exchanger assemblies) can lead to a better prioritization of the maintenance activities.

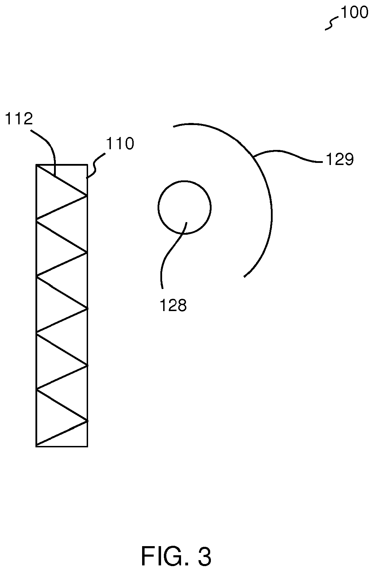

FIG. 3 shows another schematic cross section view of an air conditioning system 100, showing a protective screen 129 disposed to at least partially block UV light emitted by the UV light source 128, in accordance with various embodiments.

According to various embodiments, the UV light source includes one or more UV lamps and/or UV-LEDs.

According to various embodiments, with the use of temperature and/or humidity sensors, the state of cleanliness of the heat exchanger 112 can be monitored and thus the energy consumption of the cleaning system 120 may be reduced and life of the components of the air conditioning system 100 may be extended.

The cleaning system 120 according to various embodiments may be included in a heat exchanger assembly 110. Therefore, the present invention also concerns a heat exchanger assembly 110 including a cleaning system 120 according to various embodiments. In various embodiments, the cleaning system 120 and the heat exchanger assembly 110 include the controller 140.

The heat exchanger 112 according to various embodiments may be included in an air conditioning system 100. Therefore, the present disclosure also concerns an air conditioning system 100 including a heat exchanger 112 according to various embodiments, or a heat exchanger assembly 110 according to various embodiments.

The cleaning system may be implemented with heating and cooling applications where air-to-liquid heat exchanger assemblies are employed. The air conditioning system 100 may be, for example, an air cooling system, an air dehumidifying system, a heat pump, a variable refrigerant flow air conditioning system.

**Claims**

1. A cleaning system (120), for cleaning a heat exchanger (112) of an air conditioning system (100), the cleaning system (120) comprising a spray subsystem (122) configured to spray a cleaning liquid onto the heat exchanger (112) during a cleaning cycle, and further comprising a UV light source (128) configured to irradiate a surface of the heat exchanger (112), during the cleaning cycle, **characterised by**

   further comprising a sensor subsystem (130) for monitoring an effectiveness of the heat exchange between the heat exchanger (112) and air,
   further comprising a controller (140) configured to control the cleaning system (120) to clean the heat exchanger (112), and further configured to obtain sensor data from the sensor subsystem (130) to monitor the effectiveness, wherein the controller (140) is configured to activate a next cleaning cycle when the effectiveness has deviated from a pre-determined threshold.

2. The cleaning system (120) of claim 1, wherein the spray subsystem (122) comprises spray nozzles (123) arranged such that the cleaning liquid is sprayable onto the surface of the heat exchanger (112).

3. The cleaning system (120) of claim 2, further comprising a cleaning liquid reservoir (124) and a cleaning liquid pump (125), the cleaning liquid reservoir (124) being in fluid connection with the spray nozzles (123) via the cleaning liquid pump (125).

4. The cleaning system (120) of claim 3, further comprising a cleaning liquid collection tray (126) disposed in proximity to the heat exchanger (112) to collect the cleaning liquid, and optionally wherein the cleaning liquid collection tray (126) is configured to return the cleaning liquid to the cleaning liquid reservoir (124).

5. The cleaning system (120) of claim 1, wherein the sensor subsystem (130) comprises

   a downstream temperature sensor (132) disposed in relation to the heat exchanger (112) such that a downstream air temperature is measurable, of air which has passed through the heat exchanger (112), and
   an upstream temperature sensor (136) disposed in relation to the heat exchanger (112) such that an upstream air temperature is measurable, of air which is yet to pass through the heat exchanger (112).

6. The cleaning system (120) of claim 1 or claim 5, wherein the sensor subsystem (130) comprises

a downstream humidity sensor (134) disposed in relation to the heat exchanger (112) such that a downstream air humidity is measurable, of air which has passed through the heat exchanger (112), and
an upstream humidity sensor (138) disposed in relation to the heat exchanger (112) such that an upstream air humidity is measurable, of air which is yet to pass through the heat exchanger (112).

7. The cleaning system (120) of claim 1, wherein the controller (140) is configured to monitor the effectiveness in a continuous or in a time-discrete monitoring pattern when the air conditioning system (100) is in operation.

8. The cleaning system (120) of any of claims 1 or 7, wherein the controller (140) is configured to compare the effectiveness with a previous effectiveness, wherein the previous effectiveness is the one after a previous cleaning cycle, and wherein the controller (140) is configured to activate a next cleaning cycle when the effectiveness has deviated from the previous effectiveness by a predetermined value.

9. The cleaning system (120) of claim 8, wherein the controller (140) is configured to, based on a history log of the effectiveness, determine an effect of the cleaning cycle and thereby evaluating an estimated useful life of the cleaning system and/or of the heat exchanger (112), and wherein the history log comprises data obtained from the sensor subsystem (130).

10. The cleaning system (120) of any of the previous claims, further comprising a protective screen (129) disposed to at least partially block UV light emitted by the UV light source (128) in a direction other than towards the heat exchanger (112).

11. A heat exchanger assembly (110) for an air conditioning system (100), wherein the heat exchanger assembly (110) comprises the cleaning system (120) according to any of the previous claims.

12. An air conditioning system (100) configured to condition air by providing one or more of: heating, cooling, reducing humidity, increasing humidity; and comprising the cleaning system (120) according to any of the previous claims 1 to 10 or the heat exchanger assembly (110) according to claim 11.

13. A method for operating a cleaning system (120) according to any of the claims 1 to 10, comprising operating the spray subsystem (122) to spray a cleaning liquid onto the heat exchanger (112), and operating the UV light source (128) to irradiate a surface of the heat exchanger (112).

14. A method for determining a useful life of a heat exchanger (112) of an air conditioning system (100) comprising a cleaning system (120) according to any of claims 1 to 10, the method comprising providing a history log of effectiveness of the heat exchange between the heat exchanger (112) and air, and determining an effect of a latest cleaning cycle, and thereby evaluating an estimated useful life of the heat exchanger (112), and wherein the history log comprises data obtained from the sensor subsystem (130) for monitoring an effectiveness of the heat exchange between the heat exchanger (112) and air.

**Patentansprüche**

1. Reinigungssystem (120) zur Reinigung eines Wärmetauschers (112) einer Klimaanlage (100), wobei das Reinigungssystem (120) ein Sprühteilsystem (122) umfasst, das ausgelegt ist zum Sprühen einer Reinigungsflüssigkeit auf den Wärmetauscher (112) während eines Reinigungszyklus, und ferner umfassend eine UV-Lichtquelle (128), ausgelegt zum Bestrahlen einer Oberfläche des Wärmetauschers (112) während des Reinigungszyklus, **dadurch gekennzeichnet, dass**

es ferner ein Sensorteilsystem (130) zum Überwachen einer Effektivität des Wärmeaustauschs zwischen dem Wärmetauscher (112) und Luft umfasst,
es ferner eine Steuerung (140) umfasst, die ausgelegt ist zum Steuern des Reinigungssystems (120) zum Reinigen des Wärmetauschers (112) und ferner ausgelegt ist zum Erhalten von Sensordaten vom Sensorteilsystem (130) zum Überwachen der Effektivität,
wobei die Steuerung (140) ausgelegt ist zum Aktivieren eines nächsten Reinigungszyklus, wenn die Effektivität von einer vorbestimmten Schwelle abgewichen ist.

2. Reinigungssystem (120) nach Anspruch 1, wobei das Sprühteilsystem (122) Sprühdüsen (123) umfasst, die so angeordnet sind, dass die Reinigungsflüssigkeit auf die Oberfläche des Wärmetauschers (112) sprühbar ist.

3. Reinigungssystem (120) nach Anspruch 2, ferner umfassend einen Reinigungsflüssigkeitsbehälter (124) und eine Reinigungsflüssigkeitspumpe (125), wobei der Reinigungsflüssigkeitsbehälter (124) über die Reinigungsflüssigkeitspumpe (125) in Fluidverbindung mit den Sprühdüsen (123) ist.

4. Reinigungssystem (120) nach Anspruch 3, ferner umfassend eine Reinigungsflüssigkeitssammelschale (126), angeordnet in Nähe zum Wärmetauscher (112), zum Sammeln der Reinigungsflüssigkeit, und wobei optional die Reinigungsflüssigkeitssammelschale (126) ausgelegt ist zum Rückführen der Reinigungsflüssigkeit zum Reinigungsflüssigkeitsbehälter (124).

5. Reinigungssystem (120) nach Anspruch 1, wobei das Sensorteilsystem (130) Folgendes umfasst einen stromabwärtigen Temperatursensor (132), in Bezug zum Wärmetauscher (112) so angeordnet, dass eine stromabwärtige Lufttemperatur von Luft, die den Wärmetauscher (112) durchströmt hat, messbar ist, und einen stromaufwärtigen Temperatursensor (136), in Bezug zum Wärmetauscher (112) so angeordnet, dass eine stromaufwärtige Lufttemperatur von Luft, die den Wärmetauscher (112) noch durchströmen wird, messbar ist.

6. Reinigungssystem (120) nach Anspruch 1 oder Anspruch 5, wobei das Sensorteilsystem (130) Folgendes umfasst einen stromabwärtigen Feuchtigkeitssensor (134), in Bezug zum Wärmetauscher (112) so angeordnet, dass eine stromabwärtige Luftfeuchtigkeit von Luft, die den Wärmetauscher (112) durchströmt hat, messbar ist, und einen stromaufwärtigen Feuchtigkeitssensor (138), in Bezug zum Wärmetauscher (112) so angeordnet, dass eine stromaufwärtige Luftfeuchtigkeit von Luft, die den Wärmetauscher (112) noch durchströmen wird, messbar ist.

7. Reinigungssystem (120) nach Anspruch 1, wobei die Steuerung (140) ausgelegt ist zum Überwachen der Effektivität in einem kontinuierlichen oder zeitdiskreten Überwachungsmuster, wenn die Klimaanlage (100) in Betrieb ist.

8. Reinigungssystem (120) nach einem der Ansprüche 1 oder 7, wobei die Steuerung (140) ausgelegt ist zum Vergleichen der Effektivität mit einer vorherigen Effektivität, wobei die vorherige Effektivität diejenige nach einem vorherigen Reinigungszyklus ist, und wobei die Steuerung (140) ausgelegt ist zum Aktivieren eines nächsten Reinigungszyklus, wenn die Effektivität um einen vorbestimmten Wert von der vorherigen Effektivität abgewichen ist.

9. Reinigungssystem (120) nach Anspruch 8, wobei die Steuerung (140) ausgelegt ist zum, basierend auf einem Verlaufsprotokoll der Effektivität, Bestimmen einer Auswirkung des Reinigungszyklus und dadurch Evaluieren einer geschätzten Nutzbarkeitsdauer des Reinigungssystems und/oder des Wärmetauschers (112), und wobei das Verlaufsprotokoll vom Sensorteilsystem (130) erhaltene Daten umfasst.

10. Reinigungssystem (120) nach einem der vorhergehenden Ansprüche, ferner umfassend einen Schutzschirm (129), angeordnet, um durch die UV-Lichtquelle (128) in einer Richtung, die eine andere als die hin zum Wärmetauscher (112) ist, emittiertes UV-Licht zumindest teilweise zu blockieren.

11. Wärmetauscheranordnung (110) für eine Klimaanlage (100), wobei die Wärmetauscheranordnung (110) das Reinigungssystem (120) nach einem der vorhergehenden Ansprüche umfasst.

12. Klimaanlage (100), ausgelegt zum Klimatisieren von Luft durch Bereitstellen von einem oder mehreren aus Folgendem: Heizung, Kühlung, Verringerung der Feuchtigkeit, Erhöhung der Feuchtigkeit; und umfassend das Reinigungssystem (120) nach einem der vorhergehenden Ansprüche 1 bis 10 oder die Wärmetauscheranordnung (110) nach Anspruch 11.

13. Verfahren zum Betreiben eines Reinigungssystems (120) nach einem der Ansprüche 1 bis 10, umfassend Betreiben des Sprühteilsystems (122) zum Sprühen einer Reinigungsflüssigkeit auf den Wärmetauscher (112) und Betreiben der UV-Lichtquelle (128) zum Bestrahlen einer Oberfläche des Wärmetauschers (112).

14. Verfahren zum Bestimmen einer Nutzbarkeitsdauer eines Wärmetauschers (112) einer Klimaanlage (100), umfassend ein Reinigungssystem (120) nach einem der Ansprüche 1 bis 10, wobei das Verfahren Folgendes umfasst: Bereitstellen eines Verlaufsprotokolls von Effektivität des Wärmeaustauschs zwischen dem Wärmetauscher (112) und Luft, und Bestimmen einer Auswirkung eines letzten Reinigungszyklus und dadurch Evaluieren einer geschätzten Nutzbarkeitsdauer des Wärmetauschers (112), und wobei das Verlaufsprotokoll von dem Sensorteilsystem (130) erhaltene Daten zum Überwachen einer Effektivität des Wärmeaustauschs zwischen dem Wärmetauscher (112) und Luft umfasst.

**Revendications**

**1.** Système de nettoyage (120), pour nettoyer un échangeur de chaleur (112) d'un système de climatisation (100), le système de nettoyage (120) comprenant un sous-système de pulvérisation (122) configuré pour pulvériser un liquide de nettoyage sur l'échangeur de chaleur (112) pendant un cycle de nettoyage, et comprenant en outre une source de lumière UV (128) configurée pour irradier une surface de l'échangeur de chaleur (112), pendant le cycle de nettoyage, **caractérisé par**

le fait de comprendre en outre un sous-système de capteurs (130) pour surveiller une efficacité de l'échange de chaleur entre l'échangeur de chaleur (112) et l'air,
le fait de comprendre en outre un dispositif de commande (140) configuré pour commander le système de nettoyage (120) afin qu'il nettoie l'échangeur de chaleur (112), et configuré en outre pour obtenir des données de capteurs en provenance du sous-système de capteurs (130) pour surveiller l'efficacité,
le dispositif de commande (140) étant configuré pour activer un cycle de nettoyage suivant lorsque l'efficacité a dévié par rapport à un seuil prédéterminé.

**2.** Système de nettoyage (120) selon la revendication 1, dans lequel le sous-système de pulvérisation (122) comprend des buses de pulvérisation (123) agencées de telle sorte que le liquide de nettoyage puisse être pulvérisé sur la surface de l'échangeur de chaleur (112) .

**3.** Système de nettoyage (120) selon la revendication 2, comprenant en outre un réservoir de liquide de nettoyage (124) et une pompe à liquide de nettoyage (125), le réservoir de liquide de nettoyage (124) étant en communication fluidique avec les buses de pulvérisation (123) par l'intermédiaire de la pompe à liquide de nettoyage (125).

**4.** Système de nettoyage (120) selon la revendication 3, comprenant en outre un plateau de collecte de liquide de nettoyage (126) disposé à proximité de l'échangeur de chaleur (112) pour collecter le liquide de nettoyage, et facultativement le plateau de collecte de liquide de nettoyage (126) étant configuré pour renvoyer le liquide de nettoyage au réservoir de liquide de nettoyage (124).

**5.** Système de nettoyage (120) selon la revendication 1, dans lequel le sous-système de capteurs (130) comprend

un capteur de température aval (132) disposé relativement à l'échangeur de chaleur (112) de telle sorte qu'une température de l'air aval soit mesurable, de l'air qui a traversé l'échangeur de chaleur (112), et
un capteur de température amont (136) disposé relativement à l'échangeur de chaleur (112) de telle sorte qu'une température de l'air amont soit mesurable, de l'air qui doit encore traverser l'échangeur de chaleur (112).

**6.** Système de nettoyage (120) selon la revendication 1 ou la revendication 5, dans lequel le sous-système de capteurs (130) comprend

un capteur d'humidité en aval (134) disposé relativement à l'échangeur de chaleur (112) de telle sorte qu'une humidité de l'air en aval soit mesurable, de l'air qui a traversé l'échangeur de chaleur (112), et
un capteur d'humidité amont (138) disposé relativement à l'échangeur de chaleur (112) de telle sorte qu'une humidité de l'air amont soit mesurable, de l'air qui doit encore traverser l'échangeur de chaleur (112).

**7.** Système de nettoyage (120) selon la revendication 1, dans lequel le dispositif de commande (140) est configuré pour surveiller l'efficacité dans un motif de surveillance continu ou discret dans le temps lorsque le système de climatisation (100) est en fonctionnement.

**8.** Système de nettoyage (120) selon l'une quelconque des revendications 1 ou 7, dans lequel le dispositif de commande (140) est configuré pour comparer l'efficacité avec une efficacité précédente, l'efficacité précédente étant celle après un cycle de nettoyage précédent, et le dispositif de commande (140) étant configuré pour activer un cycle de nettoyage suivant lorsque l'efficacité a dévié par rapport à l'efficacité précédente d'une valeur prédéterminée.

**9.** Système de nettoyage (120) selon la revendication 8, dans lequel le dispositif de commande (140) est configuré pour, sur la base d'un journal d'historique de l'efficacité, déterminer un effet du cycle de nettoyage et évaluer ainsi une durée de vie utile estimée du système de nettoyage et/ou de l'échangeur de chaleur (112), et le journal d'historique comprenant des données obtenues en provenance du sous-système de capteurs (130).

10. Système de nettoyage (120) selon l'une quelconque des revendications précédentes, comprenant en outre un écran de protection (129) disposé pour bloquer au moins partiellement la lumière UV émise par la source de lumière UV (128) dans une direction autre que vers l'échangeur de chaleur (112).

11. Ensemble échangeur de chaleur (110) pour un système de climatisation (100), l'ensemble échangeur de chaleur (110) comprenant le système de nettoyage (120) selon l'une quelconque des revendications précédentes.

12. Système de climatisation (100) configuré pour conditionner de l'air en fournissant un ou plusieurs éléments parmi : chauffage, refroidissement, réduction d'humidité, augmentation de l'humidité ; et comprenant le système de nettoyage (120) selon l'une quelconque des revendications précédentes 1 à 10 ou l'ensemble échangeur de chaleur (110) selon la revendication 11.

13. Procédé de fonctionnement d'un système de nettoyage (120) selon l'une quelconque des revendications 1 à 10, comprenant le fait de faire fonctionner le sous-système de pulvérisation (122) pour qu'il pulvérise un liquide de nettoyage sur l'échangeur de chaleur (112), et le fait de faire fonctionner la source de lumière UV (128) pour qu'elle irradie une surface de l'échangeur de chaleur (112).

14. Procédé de détermination d'une durée de vie utile d'un échangeur de chaleur (112) d'un système de climatisation (100) comprenant un système de nettoyage (120) selon l'une quelconque des revendications 1 à 10, le procédé comprenant la fourniture d'un historique d'efficacité de l'échange de chaleur entre l'échangeur de chaleur (112) et l'air, et la détermination d'un effet d'un dernier cycle de nettoyage, et ainsi l'évaluation d'une durée de vie utile estimée de l'échangeur de chaleur (112), et le journal d'historique comprenant des données obtenues en provenance du sous-système de capteurs (130) pour surveiller une efficacité de l'échange de chaleur entre l'échangeur de chaleur (112) et l'air.

FIG. 1

FIG. 2

100

112

110

129

128

FIG. 3

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 100526446 B1